# FASCICULE DE BREVET EUROPEEN

(11) **EP 4 188 596 B1**
(45) Date de publication et mention de la délivrance du brevet: **18.09.2024**
(21) Numéro de dépôt: 21742140.3
(22) Date de dépôt: 13.07.2021
(51) Int. Cl.: B01J 31/00, B01J 31/02, B01J 31/12, B01J 31/14, B01J 31/22, B01J 31/18

(54) **PROCEDE D'OLIGOMERISATION D'ETHYLENE COMPRENANT LA PREPARATION IN SITU DE LA COMPOSITION CATALYTIQUE**
VERFAHREN ZUR OLIGOMERISIERUNG VON ETHYLEN UMFASSEND DIE IN-SITU HERSTELLUNG DER KATALYTISCHEN ZUSAMMENSETZUNG
ETHYLENE OLIGOMERISATION PROCESS COMPRISING IN SITU PREPARATION OF THE CATALYTIC COMPOSITION

(30) Priorité: 30.07.2020 FR 2008093
(43) Date de publication de la demande: 07.06.2023
(73) Titulaire: IFP Energies nouvelles, 92500 Rueil-Malmaison (FR)
(72) Inventeur: MICHEL, Typhène, 92852 RUEIL-MALMAISON CEDEX (FR); BREUIL, Pierre-Alain, 92852 RUEIL-MALMAISON CEDEX (FR); MAGNA, Lionel, 92852 RUEIL-MALMAISON CEDEX (FR)
(74) Mandataire: IFP Energies nouvelles
(86) Numéro de dépôt international: PCT/EP2021/069498
(87) Numéro de publication internationale: WO 2022/023028

(56) Documents cités:
- EP-A2- 0 611 743
- EP-A2- 0 668 105
- WO-A1-2006/108803
- WO-A2-02/083306
- US-A1- 2001 053 742
- US-A1- 2007 161 839
- US-A1- 2012 310 025

## Description

### DOMAINE TECHNIQUE DE L'INVENTION

L'invention concerne un procédé d'oligomérisation d'éthylène, de préférence de trimérisation sélective de l'éthylène en hexène-1, comprenant la mise en contact simultanée de l'éthylène avec les composants d'une composition catalytique à base de chrome.

### ART ANTERIEUR

La trimérisation de l'éthylène en hexène-1 par un catalyseur homogène à base de chrome est étudiée depuis les années 1980. Parmi les systèmes catalytiques connus pour conduire à la production sélective d'hexène-1, on peut citer par exemple les systèmes décrits dans les brevets US 5 198 563, US 5 288 823, US 5 382 738, EP 608 447, EP 611 743, EP 614 865. Ces catalyseurs sont préparés à partir d'un sel de chrome et d'un amidure métallique, un pyrrolure en particulier, activé in situ par un alkylaluminium ou un mélange d'alkylaluminium et de chloroalkylaluminium.

Il est couramment observé lors de l'opération de ces systèmes catalytiques à base de chrome que l'activité et la productivité du système catalytique peuvent diminuer. En outre, des conditions de réaction dures, telles que des températures élevées, peuvent augmenter la productivité du catalyseur, mais cela conduit généralement à une durée de vie plus courte du système catalytique et parfois même à une perte de la sélectivité initiale du catalyseur. L'utilisation d'additifs peut alors être envisagée pour contrebalancer ce phénomène et assurer la bonne stabilité du catalyseur et le maintien de ses performances notamment à haute température.

Le document US2001/0053742 décrit l'effet de l'ordre d'addition des différents constituants d'une composition catalytique comprenant du chrome, un dérive de pyrrole et un alkylaluminium sur la formation de polyéthylène lors de la réaction de trimérisation de l'éthylène. Ce document précise que la mise en contact des différents constituants lors de la préparation de la composition catalytique doit être réalisée sous atmosphère inerte et à une température comprise entre 10 et 40°C de manière à minimiser la formation de particules et à améliorer les performances de la composition catalytique ainsi préparée.

Etonnamment, la demanderesse a découvert un nouveau procédé d'oligomérisation de l'éthylène s'affranchissant des inconvénients liés à la préparation préalable de la composition catalytique et permettant d'obtenir d'excellentes performances en termes de sélectivité et d'activité. En particulier, ledit procédé comprend la mise en contact des différents composants de la composition catalytique *in situ* c'est-à-dire dans le réacteur en présence d'éthylène à une température comprise entre 100 et 190°C.

### OBJET DE L'INVENTION

La présente invention concerne un procédé d'oligomérisation d'éthylène tel que défini dans les revendications annexées comprenant, à une température comprise entre 90 et 190°C, la mise en contact simultanée de l'éthylène avec les composants suivants :
- un précurseur métallique de chrome
- un dérivé pyrrole
- un composé à base d'aluminium de formule générale AIR²R³R⁴ dans laquelle les groupements R², R³ et R⁴, identiques ou différents, sont choisis indépendamment parmi un hydrogène, les groupements alkyles en C₁-C₂₀, alkoxy en C₁-C₂₀ et aryloxy en C₅-C₃₀,
- au moins un composé halogéné d'aluminium répondant à la formule générale AlₙR⁵ₒYₚ, dans laquelle R⁵ est un groupement alkyle en C₁-C₂₀, Y est un halogène, n est un nombre entier de 1 à 2, o un nombre entier de 1 à 3 et p un nombre entier de 1 à 3 ;
- un additif aromatique ;
- optionnellement un solvant,
lesdits composants formant *in situ* une composition catalytique.Le procédé selon la présente invention permet avantageusement de former *in situ* la composition catalytique à haute température et en présence d'éthylène.

Un avantage du procédé selon la présente invention est d'améliorer significativement la stabilité de la formulation catalytique à haute température tout en maintenant une forte activité et une forte sélectivité en faveur de l'hexène-1.

De préférence, le composé à base d'aluminium et le composé halogéné d'aluminium sont mélangés préalablement à leur mise en contact avec l'éthylène.

De préférence, le composé à base d'aluminium et le composé halogéné d'aluminium sont mélangés avant d'être mélangés avec le dérivé pyrrole préalablement à leur mise en contact avec l'éthylène.

De préférence, le composé à base d'aluminium et le composé halogéné d'aluminium, le dérivé pyrrole et l'additif aromatique sont mélangés préalablement à leur mise en contact avec l'éthylène.

De préférence, le précurseur métallique de chrome et l'additif aromatique sont mélangés préalablement à leur mise en contact avec l'éthylène.

De préférence, tous les composants ne sont pas mélangés avant d'être mis en contact avec l'éthylène. Dans ce mode de réalisation, tous les composants sont mélangés lors de leurs mises en contact avec l'éthylène.

De préférence, la température de mise en contact des composants avec l'éthylène est comprise entre 95 et 185°C, de préférence entre 100 et 160°C.

De préférence, l'éthylène est en mélange avec de l'hydrogène.

Selon l'invention, le dérivé pyrrole répond à la formule générale (I) 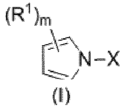 dans laquelle
- R¹ est choisi indépendamment parmi un halogène, un groupement alkyle en C₁-C₁₅, un groupement C(O)R', un groupement COOR", CCl₃, CF₃, R' étant choisi parmi H, un alkyl en C₁-C₆, un chlore, un brome, un fluor ; R" étant choisi parmi H, un alkyl en C₁-C₆;
- m est un entier compris entre 0 et 4 ;
- X est choisi parmi un hydrogène, un lithium, un sodium, un potassium, un césium, ou un atome d'aluminium.

Selon l'invention , le rapport molaire entre le nombre total d'atome d'halogène apporté par les composants de la composition catalytique et la somme des atomes d'aluminium, noté Altot, apportés par le composé à base d'aluminium et le composé halogéné d'aluminium, noté halo/Altot, est compris entre 0,10 et 3,0, de préférence entre 0,15 et 2,5, de préférence entre 0,20 et 2,0.

De préférence, l'additif aromatique est choisi parmi un éther aromatique et/ou un hydrocarbure aromatique.

De préférence, l'éther aromatique réponds à la formule générale (II) suivante : 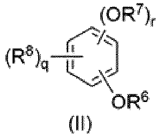 dans laquelle
- R₆ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R₇ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R⁸ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- q est un nombre entier compris 0 et 4,
- r est un nombre entier égale à 0 ou 1.

De préférence, le rapport molaire entre l'éther aromatique et le précurseur métallique à base de chrome, noté éther aromatique/Cr, est compris entre 0,5 et 2000,0, de préférence entre 1,0 et 800,0, de préférence entre 2,0 et 600,0, de préférence entre 3,0 et 400,0.

De préférence, l'hydrocarbure aromatique répond à la formule générale (III) suivante 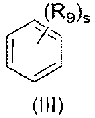 dans laquelle
- R⁹ est choisi indépendamment parmi un groupement alkyle en C₁-C₂₀, un groupement cycloalkyle en C₃-C₂₀,
- s est un entier compris entre 0 et 6.

De préférence, le rapport molaire entre l'hydrocarbure aromatique et le précurseur métallique à base de chrome, noté hydrocarbure aromatique/Cr, est compris entre 5,0 et 6000,0, de préférence entre 10,0 et 5500,0, de préférence entre 15,0 et 5000,0.

### DEFINITIONS ET ABREVIATIONS

Il est précisé que, dans toute cette description, les expressions « compris(e) entre ... et ... » « comprenant entre ... et ... » doivent s'entendre comme incluant les bornes citées.

L'expression « en Cx-Cy » pour un groupement hydrocarboné signifie que ledit groupement comprend x à y atomes de carbone.

On entend par groupement alkyle en Cx-Cy, une chaine hydrocarbonée comprenant entre x et y atomes de carbone, alkyle en Cₓ-C_{y}, par exemple entre 1 et 20 atomes de carbone noté alkyle en C₁-C₂₀, linéaire ou ramifiée, non cyclique, cyclique ou polycyclique, substituée ou non. Par exemple, on entend par alkyle en C₁-C₈, un alkyle choisi parmi les groupements méthyle, éthyle, propyle, butyle, pentyle, cyclopentyle, hexyle, cyclohexyle, heptyle, octyle.

On entend par alkoxy, un groupement monovalent constitué d'un groupement alkyle lié à un atome d'oxygène tel que les groupements CH₃O-, C₂H₅O-, C₃H₇O-.

On entend par aryloxy, un groupement monovalent constitué d'un groupement aryle lié à un atome d'oxygène tel que le groupement C₆H₅O-.

On entend par groupement alkényle, un groupement hydrocarboné comprenant au moins une double liaison, ledit groupement étant linéaire ou ramifié et comportant de 2 à 20 atomes de carbone, de préférence de 2 à 6 atomes de carbone, par exemple le groupement éthényle, vinyle, butényle ou 2-propène-1-yl (allyle).

On entend par groupement aralkyle, un groupement comprenant un groupement alkyle dont un atome d'hydrogène est substitué par un groupement aryle, les groupements alkyle et aryle étant tels que définis ci-dessus.

On entend par groupement aryle, un groupement aromatique, mono ou polycyclique, fusionné ou non, substitué ou non, comprenant entre 5 et 30 atomes de carbone, noté aryle en C₅-C₃₀. On entend par Cr(lll) un composé à base de chrome à un degré d'oxydation +III. De manière similaire, on entend par Cr(ll) et Cr(I) un composé à base de chrome à un degré d'oxydation respectivement de ₊II et +I.

Les rapports molaires cités dans la présente invention notamment par rapport au précurseur de chrome sont entendus et exprimés par rapport au nombre de moles de chrome contenues dans la composition catalytique.

### DESCRIPTION DETAILLEE DE L'INVENTION

Dans le sens de la présente invention, les différents modes de réalisation présentés peuvent être utilisés seuls ou en combinaison les uns avec les autres, sans limitation de combinaison. Dans le sens de la présente invention, les différentes plages de paramètres pour une étape donnée telles que les plages de pression et les plages de température peuvent être utilisées seules ou en combinaison. Par exemple, dans le sens de la présente invention, une plage de valeur préférée de pression peut être combinée avec une plage de valeur de température plus préférée.

La présente invention concerne un procédé d'oligomérisation d'éthylène tel que définit dans les revendications annexées comprenant, à une température comprise entre 90 et 190°C, la mise en contact simultanée de l'éthylène avec les composants suivants :
- un précurseur métallique de chrome
- un dérivé pyrrole
- un composé à base d'aluminium de formule générale AIR²R³R⁴ dans laquelle les groupements R², R³ et R⁴, identiques ou différents, sont choisis indépendamment parmi un hydrogène, les groupements alkyles en C₁-C₂₀, alkoxy en C₁-C₂₀ et aryloxy en C₅-C₃₀,
- au moins un composé halogéné d'aluminium répondant à la formule générale AlₙR⁵ₒYₚ, dans laquelle R⁵ est un groupement alkyle en C₁-C₂₀, Y est un halogène, n est un nombre entier de 1 à 2, o un nombre entier de 1 à 3 et p un nombre entier de 1 à 3 ;
- un additif aromatique ;
- optionnellement un solvant,
lesdits composants formant *in situ* une composition catalytique.

Un avantage du procédé selon l'invention est de simplifier la mise en oeuvre du procédé d'oligomérisation par une mise en oeuvre dite *in situ* dans laquelle les différents composants de la composition sont mis en contact à la température de réaction d'oligomérisation et en présence d'éthylène, sans précaution particulière.

Ainsi, le procédé selon l'invention permet de simplifier le schéma procédé et donc de limiter les coûts associés à la mise en oeuvre du procédé d'oligomérisation tout en conservant une activité et une sélectivité élevées.

Un autre avantage du procédé selon l'invention est de limiter la formation de polymère correspondant aux composés (C12+) ayant un nombre de carbone supérieur ou égal à 12, tel que le polyéthylène.

Un autre avantage du procédé selon l'invention est de permettre de s'affranchir des contraintes liées à la préparation et au stockage éventuel de la composition catalytique.

Les composants de la composition catalytique sont de préférence mis en contact avec l'éthylène, seuls ou en combinaison, avantageusement selon les modes de réalisations ci-après.

Dans un mode de réalisation préféré, le composé à base d'aluminium et le composé halogéné d'aluminium sont mélangés préalablement à leur mise en contact avec l'éthylène, de préférence lors de leur introduction dans un réacteur dans lequel est mis en oeuvre le procédé d'oligomérisation selon l'invention.

Dans un mode de réalisation préféré, le dérivé pyrrole est mélangé avec le composé à base d'aluminium et/ou le composé halogéné d'aluminium préalablement à leur mise en contact avec l'éthylène, de préférence lors de leur introduction dans le réacteur.

Dans un mode de réalisation préféré, le composé à base d'aluminium et le composé halogéné d'aluminium sont mélangés avant d'être mélangés avec le dérivé pyrrole, de préférence en présence d'un solvant, préalablement à leur mise en contact avec l'éthylène, de préférence lors de leur introduction dans le réacteur.

Dans un mode de réalisation préféré, le composé à base d'aluminium et le composé halogéné d'aluminium, le dérivé pyrrole et l'additif aromatique, de préférence en présence d'un solvant, sont mélangés préalablement à leur mise en contact avec l'éthylène, de préférence lors de leur introduction dans le réacteur.

Dans un mode de réalisation préféré, le précurseur métallique de chrome est mélangé avec l'additif aromatique préalablement à leur mise en contact avec l'éthylène, de préférence lors de leur introduction dans le réacteur.

De préférence, le composé à base d'aluminium et le composé halogéné d'aluminium sont introduits séparément du précurseur métallique de chrome pour leur mise en contact simultanée avec l'éthylène. En d'autres termes, le composé à base d'aluminium et/ou le composé halogéné d'aluminium ne sont pas mélangés avec le précurseur métallique de chrome préalablement à leur mise en contact avec l'éthylène.

Avantageusement, tous les composants de la composition catalytique ne sont pas mélangés avant d'être mis en contact avec l'éthylène, c'est-à-dire que tous les composants sont mélangés lors de leurs mises en contact avec l'éthylène.

De préférence, le procédé est mis en oeuvre à une pression comprise entre de 0,5 à 15 MPa, de préférence de 1,0 à 10,0 MPa, de manière préférée entre 1,5 et 8,0 MPa et de manière très préférée entre 2,0 et 7,0 MPa.

De préférence, la température de mise en contact des composants de la composition catalytique avec l'éthylène est comprise entre 95 et 185°C, de préférence entre 100 et 160°C, de manière préférée entre 105 et 155°C, de manière préférée entre 110 et 150°C, et de manière préférée entre 125 et 145°C.

Avantageusement, le procédé d'oligomérisation est mis en oeuvre à une température comprise entre 95 et 185°C, de préférence entre 100 et 160°C, de manière préférée entre 105 et 155°C, de manière préférée entre 110 et 150°C, et de manière préférée entre 125 et 145°C.

Dans un mode de réalisation préféré, la température de mise en contact de la composition catalytique dans le réacteur est identique à la température du procédé d'oligomérisation.

Avantageusement, la concentration en Cr mise en oeuvre dans le procédé d'oligomérisation par rapport au volume total de liquide mis en oeuvre dans le réacteur d'oligomérisation est comprise entre 10⁻¹² et 1 mol/L, et de préférence entre 10⁻⁹ et 0.4 mol/L.

Avantageusement, l'éthylène mis en oeuvre dans le procédé d'oligomérisation est en mélange avec de l'hydrogène. De préférence, l'hydrogène est introduit dans un ratio compris entre à 0,1 et 5,0 % poids par rapport à l'éthylène introduit, de préférence entre 0,2 et 4,0 % poids, de préférence entre 0,4 et 3,0 % poids, et de manière très préférée entre 0,5 et 2,0 % poids. Avantageusement, l'emploi d'hydrogène en mélange avec l'éthylène permet d'augmenter la productivité et de limiter le poids moléculaire des polymères (C12+).

Avantageusement, le procédé d'oligomérisation est un procédé de trimérisation de l'éthylène, de préférence pour la production d'hex-1-ène.

Avantageusement, le procédé d'oligomérisation est mis en oeuvre en continu.

La chaleur engendrée par la réaction peut être éliminée par tous les moyens connus de l'homme du métier.

La composition catalytique peut être neutralisée en aval du réacteur par tout moyen connu par l'homme du métier.

De préférence la composition catalytique dont les composants sont mis en contact avec l'éthylène dans le procédé d'oligomérisation comprend, et de préférence est constitué de :
- un précurseur métallique à base de chrome,
- un dérivé pyrrole répondant à la formule générale (I) 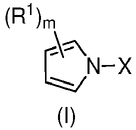 dans laquelle
   * R¹ est choisi indépendamment parmi un halogène, un groupement alkyle en C₁-C₁₅, un groupement C(O)R', un groupement COOR", CCl₃, CF₃, R' étant choisi parmi H, un alkyl en C₁-C₆, un chlore, un brome, un fluor ; R" étant choisi parmi H, un alkyl en C₁-C₆;
   * m est un entier compris entre 0 et 4 ;
   * X est choisi parmi un hydrogène, un lithium, un sodium, un potassium, un césium, ou un atome d'aluminium,
- un composé à base d'aluminium de formule générale AIR²R³R⁴ dans laquelle les groupements R², R³ et R⁴, identiques ou différents, sont choisis indépendamment parmi un hydrogène, les groupements alkyles en C₁-C₂₀, alkoxy en C₁-C₂₀ et aryloxy en C₅-C₃₀,
- un composé halogéné d'aluminium répondant à la formule générale AlₙR⁵ₒYₚ dans laquelle R⁵ est un groupement alkyle en C₁-C₂₀, Y est un halogène, n est un nombre entier de 1 à 2, o un nombre entier de 1 à 3 et p un nombre entier de 1 à 3 ;
- un additif aromatique
- éventuellement d'un solvant.

### Précurseur métallique à base de chrome

L'un des composants de la composition catalytique mis en contact avec l'éthylène dans le procédé selon l'invention est un précurseur métallique à base de chrome, de préférence choisi parmi un sel de chrome(ll) ou de chrome(III).

De préférence, le précurseur métallique à base de chrome peut comporter un ou plusieurs anions identiques ou différents, de préférence choisis dans le groupe formé par les halogénures, les carboxylates, les acétylacétonates, les anions alkoxy et aryloxy. Le composé du chrome peut être un sel de chrome(ll) ou de chrome(III), mais aussi un sel de degré d'oxydation différent pouvant comporter un ou plusieurs anions identiques ou différents tels que par exemple des halogénures, des carboxylates, des acétylacétonates, des anions alkoxy ou aryloxy.

De préférence, les anions halogénures sont choisis parmi le chlorure, de bromure, de fluorure ou d'iodure.

De préférence, les anions carboxylates sont choisis parmi les carboxylates ayant une chaine alkyle linéaire ou ramifié en C₃-C₂₀, de préférence en C₃-C₁₅, de préférence en C₄-C₁₂, de préférence C₅-C₁₀, de préférence ladite chaine alkyle est substituée ou non par un ou plusieurs atomes de fluor, de chlore ou de brome.

De préférence, les anions alkoxy sont choisis parmi les alkoxy ayant une chaine alkyle en C₁-C₂₀, linéaire, ramifiée, cyclique ou non-cyclique, de préférence en C₂-C₁₅, de préférence en C₃-C₁₂, de préférence C₄-C₁₀, de préférence ladite chaine alkyle est substituée ou non par un ou plusieurs atomes de fluor, de chlore ou de brome.

De préférence, les anions aryloxy sont choisis parmi les aryloxy ayant un groupement aryl en C₅-C₃₀, de préférence en C₅-C₂₀, de préférence en C₆-C₁₅, de préférence C₆-C₁₂, de préférence ledit groupement aryle est substitué ou non par un ou plusieurs atomes de fluor, de chlore ou de brome.

De préférence, le précurseur métallique à base de chrome peut être un composé du chrome(lll) mais un composé du chrome(I) ou du chrome(ll) peut aussi convenir. A titre d'exemples non limitatifs on peut citer l'acetylacetonate de Cr(III), le trifluoro-acetylacetonate de Cr(III), l'hexafluoroacetylacetonate de Cr(III), l'acetate de Cr(III), le 2-ethylhexanoate de Cr(III), l'heptanoate de Cr(III), le naphthenate de Cr(III), le chlorure de Cr(III), le bromure de Cr(III), pris seuls ou en mélange, pur ou dilués. Les précurseurs métalliques à base de chrome préférés sont l'acetylacetonate de Cr(III), le 2-ethylhexanoate de Cr(lll) et l'heptanoate de Cr(III).

### Dérivé de pyrrole

L'un des composants de la composition catalytique mis en contact avec l'éthylène dans le procédé selon l'invention est un dérivé pyrrole, répondant à la formule générale (I) 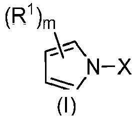 dans laquelle
- R¹ est choisi indépendamment parmi un halogène, un groupement alkyle en C₁-C₁₅, un groupement C(O)R', un groupement COOR", CCl₃, CF₃, R' étant choisi parmi H, un alkyl en C₁-C₆, un chlore, un brome, un fluor ; R" étant choisi parmi H, un alkyl en C₁-C₆;
- m est un entier compris entre 0 et 4 ;
- X est choisi parmi un hydrogène, un lithium, un sodium, un potassium, un césium, ou un atome d'aluminium.

Avantageusement, R¹ est choisi indépendamment parmi un fluor, un chlore, un groupement alkyle en C₁-C₁₀, un groupement C(O)R', un groupement COOR", CCl₃, CF₃, R' étant choisi parmi H, un méthyle, un éthyle, un propyle, un butyle, un pentyl, un cyclohexyl, un chlore, un fluor. De manière préférée, R¹ est choisi indépendamment parmi un fluor, un chlore, un groupement alkyle en C₁-C₆, un groupement C(O)R', un groupement COOR", CCl₃, CF₃, R' étant choisi parmi H, un méthyle, un éthyle, un propyle, un butyle, R" étant choisi parmi H, méthyle, un éthyle, un propyle, un butyle.

De préférence, m est un entier égal à 0, 1, 2, 3 ou 4, de manière préférée m est égale à 0 ou 2.

De préférence, le dérivé de pyrrole est choisi parmi tétrahydroindole, 2,5-diméthylpyrrole, 3,4-diméthylpyrrole, 3,4-dichloro-pyrrole, 2,3,4,5-tétrachloro-pyrrole, 2,4-diméthyl-3-éthylpyrrole, acide pyrrole-2-carboxylique, 2-acetylpyrrole, pyrrole-2-carboxaldéhyde, 3-acétyl-2,4-diméthylpyrrole, éthyl-2,4-diméthyl-5-(éthoxycarbonyl)-3-pyrroleproprionate, éthyl-3,5-diméthyl-2-pyrrolecarboxylate, seuls ou en mélanges. De manière préférée, le dérivé du pyrrole est choisi parmi le pyrrole (C₄H₅N) et le 2, 5-diméthylpyrrole.

De préférence, le rapport molaire du composé dérivé du pyrrole sur le précurseur métallique à base de chrome, noté DMP/Cr, est compris entre 0,5 et 40,0, de préférence entre 1,0 et 10,0, de préférence entre 1,5 et 8,0, de manière préférée entre 2,0 et 5,0 et de manière très préférée entre 2,5 et 4,5.

### Composé à base d'aluminium

L'un des composants de la composition catalytique mis en contact avec l'éthylène dans le procédé selon l'invention est un composé à base d'aluminium de formule générale AIR²R³R⁴ dans laquelle les groupements R², R³ et R⁴, identiques ou différents, sont choisis indépendamment parmi un hydrogène, les groupements alkyles en C₁-C₂₀, alkoxy en C₁-C₂₀ et aryloxy en C₅-C₃₀.

Lorsque au moins un des groupements R², R³ et R⁴ est choisi parmi les groupements alkyle et alkyloxy, lesdits groupements alkyle et alkyloxy comprennent de préférence entre 1 et 15 atomes de carbone, de préférence entre 1 et 10 atomes de carbone, de manière préférée entre 1 et 6 atomes de carbone et de manière très préférée entre 1 et 4 atomes de carbone.

Lorsque au moins un des groupements R², R³ et R⁴ est choisi parmi les aryloxy, lesdits groupements aryloxy comprennent de préférence entre 5 et 20 atomes de carbone, de préférence entre 5 et 15 atomes de carbone et de manière préférée entre 5 et 10 atomes de carbone.

De préférence, un des groupements R², R³ et R⁴ est un hydrogène.

De préférence, les groupements R², R³ et R⁴ sont identiques.

De préférence, au moins un des groupements R², R³ et R⁴ est choisi indépendamment des autres parmi les groupements alkyles et alkyloxy. De manière préféré, lesdits groupements alkyles et alkyloxy sont choisis parmi méthyle, éthyle, propyle, n-butyle, isobutyle, sec-butyl, tert-butyle, pentyle, hexyle, heptyle, octyle, et parmi les groupements alkyloxy correspondants.

De préférence, au moins un des groupements R², R³ et R⁴ est choisi indépendamment des autres parmi les groupements aryloxy. De manière très préférée, ledit groupement aryloxy est le phenoxy (C₆H₅0-).

Avantageusement, le ou les composés à base d'aluminium de formule générale AIR²R³R⁴ sont choisis parmi le triméthylaluminium, le triéthylaluminium, le tri-n-propylaluminium, le triisopropylaluminium, le tri-n-butylaluminium, le triisobutylaluminium, le tri-tert-butylaluminium, le trihexylaluminium, le trioctylaluminium, le diéthyléthoxyaluminium et le diméthyléthoxyaluminium. De manière préférée, le composé à base d'aluminium est le triéthylaluminium ou le triisobutylaluminium.

De préférence, le rapport molaire du composé à base d'aluminium de formule générale AIR²R³R⁴ sur le précurseur métallique à base de chrome, noté Al/Cr, est compris entre 1,0 et 100,0, de préférence entre 2,0 et 90,0, de préférence entre 3,0 et 80,0, de préférence entre 4,0 et 60,0, de préférence entre 5,0 et 50,0, de préférence entre 6,0 et 40,0, de manière préférée entre 7,0 et 30,0, de manière très préférée entre 8,0 et 20,0, et de manière encore plus préférée entre 9,0 et 15,0.

### Composé halogéné d'aluminium

L'un des composants de la composition catalytique mis en contact avec l'éthylène dans le procédé selon l'invention est un composé halogéné d'aluminium répondant à la formule générale AlₙR⁵ₒYₚ dans laquelle
- R⁵ est un groupement alkyle en C₁-C₂₀,
- Y est un halogène,
- n est un nombre entier de 1 à 2,
- o un nombre entier de 1 à 3 et
- p un nombre entier de 1 à 3.

De préférence, R⁵ est un alkyl en C₁-C₁₅, de préférence en C₁-C₁₀, de préférence en C₁-C₆. De manière préféré, R⁵ est choisi parmi méthyle, éthyle, propyle, n-butyle, isobutyle, sec-butyl, tert-butyle, pentyle, hexyle, heptyle, octyle.

De préférence, Y est choisi parmi un fluor, un chlore ou un brome. De manière préférée, Y est un chlore ou un brome.

De manière avantageuse, le composé halogéné d'aluminium est choisi dans le groupe formé par le dichlorure de méthylaluminium (MeAlCl₂), le dichlorure d'éthylaluminium (EtAlCl₂), le sesquichlorure d'éthylaluminium (Et₃Al₂Cl₃), le chlorure de diéthylaluminium (Et₂AlCl), le chlorure de diisobutylaluminium (iBu₂AlCl), le dichlorure d'isobutylaluminium (iBuAlCl₂), pris seuls ou en mélange. De manière très préférée, le composé halogéné d'aluminium est le dichlorure d'éthylaluminium (EtAlCl₂) et le chlorure de diéthylaluminium (Et₂AlCl).

De préférence, le rapport molaire du composé halogéné d'aluminium de formule générale AlₙR⁵ₒYₚsur le précurseur métallique à base de chrome, noté AIY/Cr, est compris entre 1,0 et 100,0, de préférence entre 1,5 et 80,0, de préférence entre 2,0 et 60,0, de préférence entre 2,5 et 50,0, de préférence entre 3,0 et 40,0, de préférence entre 3,5 et 30,0, de manière préférée entre 4,0 et 20,0, de manière très préférée entre 4,5 et 15,0, et de manière encore plus préférée entre 5,0 et 12,0.

Selon l'invention, le rapport molaire entre le nombre d'atome total d'halogène apporté par les composants de la composition catalytique, de préférence de chlore ou brome, et la somme des atomes d'aluminium (noté Altotal ou Altot) apportés par le composé à base d'aluminium (AI) et le composé halogéné d'aluminium (AIY), noté halo/Altot, et en particulier Cl/Altot lorsque l'halogène est le chlore et Br/Altot lorsque l'halogène est le brome, est compris entre 0,10 et 3,0, de préférence entre 0,15 et 2,5, de préférence entre 0,20 et 2,0, de manière préférée entre 0,25 et 1,5 et de manière très préférée entre 0,30 et 1,0.

Avantageusement, lorsque la composition catalytique présente des rapports molaire Halo/AI compris dans les gammes définies précédemment, ladite composition présente une meilleure activité et sélectivité en héxène-1 pour la trimérisation de l'éthylène.

### Additif aromatique

L'un des composants de la composition catalytique mis en contact avec l'éthylène dans le procédé selon l'invention est au moins un additif aromatique. Avantageusement, l'additif aromatique est un éther aromatique et/ou un hydrocarbure aromatique. De préférence, l'additif aromatique est choisi parmi les composés répondant aux formules générales Il et/ou (III) suivantes.

L'additif aromatique peut être un éther aromatique répondant à la formule générale (II) suivante : 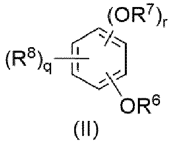 dans laquelle
- R₆ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R₇ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R⁸ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- q est un nombre entier compris 0 et 4,
- r est un nombre entier égale à 0 ou 1.

De préférence, R₆ est choisi parmi un groupement alkyl en C₁-C₁₀ un groupement cycloalkyle en C₃-C₁₀, un groupement alkényle en C₂-C₁₀, un groupement aryle en C₅-C₁₅. De préférence, R₆ est choisi parmi un groupement alkyl en C₁-C₆, un groupement cycloalkyle en C₃-C₆, un groupement alkényle en C₂-C₈, un groupement aryle en C₅-C₁₂. De manière préférée, R₆ est choisi parmi les groupements méthyl, éthyl, n-propyl, isopropyl, n-butyl, tert-butyl, pentyl, cyclohexyl, benzyl, phényl, 2-méthylphényl, 2,6-diméthylphényl ou 2,4,6-triméthylphényl. De manière très préférée, R₆ est choisi parmi les groupements méthyl, éthyl, n-propyl, isopropyl, n-butyl, tert-butyl, cyclohexyl, benzyl, phényl, 2-méthylphényl, 2,6-diméthylphényl ou 2,4,6-triméthylphényl.

De préférence, R₇ est choisi parmi un groupement alkyl en C₁-C₁₀ un groupement cycloalkyle en C₃-C₁₀, un groupement alkényle en C₂-C₁₀, un groupement aryle en C₅-C₁₅. De préférence, R₇ est choisi parmi un groupement alkyl en C₁-C₆, un groupement cycloalkyle en C₃-C₆, un groupement alkényle en C₂-C₈, un groupement aryle en C₅-C₁₂. De manière préférée, R₇ est choisi parmi un groupement méthyl, éthyl, n-propyl, isopropyl, n-butyl, tert-butyl, pentyl, cyclohexyl, benzyl, phényl, 2-méthylphényl, 2,6-diméthylphényl ou 2,4,6-triméthylphényl. De manière très préférée, R₇ est choisi parmi un groupement méthyl, éthyl, n-propyl, isopropyl, n-butyl, tert-butyl, cyclohexyl, benzyl, phényl, 2-méthylphényl, 2,6-diméthylphényl ou 2,4,6-triméthylphényl.

De préférence, R⁸ est choisi parmi un groupement alkyl en C₁-C₁₀ un groupement cycloalkyle en C₃-C₁₀, un groupement alkényle en C₂-C₁₀, un groupement aryle en C₅-C₁₅. De préférence, R⁸ est choisi parmi un groupement alkyl en C₁-C₆, un groupement cycloalkyle en C₃-C₆, un groupement alkényle en C₂-C₈, un groupement aryle en C₅-C₁₂. De manière préférée, R⁸ est choisi parmi un groupement méthyl, éthyl, n-propyl, isopropyl, n-butyl, tert-butyl, pentyl, cyclohexyl, benzyl, phényl, 2-méthylphényl, 2,6-diméthylphényl ou 2,4,6-triméthylphényl. De manière très préférée, R⁸ est choisi parmi un groupement méthyl, éthyl, n-propyl, isopropyl, n-butyl, tert-butyl, cyclohexyl, benzyl, phényl, 2-méthylphényl, 2,6-diméthylphényl ou 2,4,6-triméthylphényl.

De manière préférée, q est égale à 0, 1 ou 2.

Avantageusement, r est égale à 0 ou 1. De manière préférée, r est égale à 0.

Lorsque r est égale à 1, le groupement OR⁷ est en position, ortho, méta ou para par rapport au groupement OR⁶. De préférence, le groupement OR⁷ est en position ortho.

Lorsque q est égale à 1 ou 2, le ou les groupement R⁸, identiques ou différents, sont en position ortho, méta ou para par rapport au groupement OR⁶. De préférence, le ou les groupements R⁸ sont en position ortho.

De préférence, l'additif aromatique de type éther est choisi parmi le méthoxybenzène (ou anisole), le 2-méthylanisole, le 3-méthylanisole, le 4-méthylanisole, le 2-chloroanisole, le 3-chloroanisole, le 4-chloroanisole, le 3,5-dichloroanisole, le 2,6-dichloroanisole, le 1,2-diméthoxybenzène, le 1,3-diméthoxybenzène, le 1,4-diméthoxybenzène, le 2,3-diméthylanisole, l'éthoxybenzène, le diphényléther, le 1-méthoxynaphtalene, le 2-méthoxynaphtalene, le 2,7-diméthoxynaphtalene, le 1,3-diméthoxynaphtalene. De préférence l'additif aromatique de type éther est le méthoxybenzène, le 1,2-diméthoxybenzène et le 2,3-diméthylanisole.

De préférence, le rapport molaire entre l'éther aromatique et le précurseur métallique à base de chrome, noté éther aromatique/Cr, est compris entre 0,5 et 2000,0, de préférence entre 1,0 et 800,0, de préférence entre 2,0 et 600,0, de préférence entre 3,0 et 400,0, de préférence entre 5,0 et 300,0, de préférence entre 7,0 et 200,0, de manière préférée entre 10,0 et 150,0, de préférence entre 12,0 et 100,0, de manière préférée entre 15,0 et 90,0, manière plus préférée entre 20,0 et 80,0 et de manière encore plus préférée entre 30,0 et 70,0.

L'additif aromatique peut être un hydrocarbure aromatique répondant à la formule générale (III) suivante 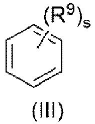 dans laquelle
- R⁹ est choisi indépendamment parmi un groupement alkyle en C₁-C₂₀, un groupement cycloalkyle en C₃-C₂₀,
- s est un entier compris entre 0 et 6.

De préférence, R⁹ est choisi parmi un groupement alkyle en C₁-C₁₀ , de préférence un groupement alkyle en C₁-C₆, un groupement cycloalkyle en C₃-C₁₀, de préférence un groupement cycloalkyle en C₃-C₆. De manière préférée R⁹ est choisi parmi des groupements méthyl, éthyl, n-propyl, isopropyl, n-butyl, tert-butyl, pentyl, cyclohexyl.

De préférence, s est égal à 0, 1, 2, 3, 4, 5 ou 6. De manière préférée, s est compris entre 1 et 4, et de manière plus préférée s est égal à 1, 2 ou 3.

De manière préférée, l'additif hydrocarbure aromatique est choisi parmi le benzène, le toluène, l'éthylbenzène, le diéthylbenzène, l'ortho-xylène, le méta-xylène, le para-xylène, le styrène, le cumène, seul ou en mélange. De préférence l'additif hydrocarbure aromatique est choisi parmi le toluène, l'éthylbenzène et l'o-xylène.

Dans un mode de réalisation particulier, la composition catalytique mise en oeuvre dans un procédé selon l'invention peut comprendre au moins un éther aromatique de formule (II) et au moins un hydrocarbure aromatique de formule (III) comme additifs.

De préférence, le rapport molaire entre l'additif hydrocarbure aromatique et le précurseur métallique à base de chrome, noté hydrocarbure aromatique/Cr, est compris entre 5,0 et 6000,0, de préférence entre 10,0 et 5500,0, de préférence entre 15,0 et 5000,0, de préférence entre 20,0 et 4500,0, de préférence entre 25,0 et 4000,0, de préférence entre 30,0 et 3500,0, de manière préférée entre 35,0 et 3000,0, de préférence entre 40,0 et 2500,0, de préférence entre 45,0 et 2000,0, m de préférence entre 50,0 et 1700,0, de préférence entre 55 et 1500,0, de préférence entre 60,0 et 1200,0, de préférence entre 70,0 et 1000,0, de préférence entre 80,à et 900,0, de préférence entre 90,0 et 800, de manière préférée entre 100 et 700, de manière préféré entre 150 et 600, et de manière très préférée entre 200 et 500.

### Solvant optionnel

L'un des composants de la composition catalytique mis en contact avec l'éthylène dans le procédé selon l'invention peut en outre être au moins un solvant. De préférence ledit solvant est choisi parmi les solvants organiques et en particulier parmi les hydrocarbures saturés, insaturés, cycliques ou non.

Le (ou les) solvant(s) est(sont) avantageusement choisi(s) parmi les solvants halogénés et les hydrocarbures, saturés ou insaturés, cycliques ou non, comprenant entre 1 et 20 atomes de carbone, de préférence entre 1 et 15 atomes de carbone et de préférence entre 4 et 15 atomes de carbone.

De préférence, le solvant est choisi parmi le pentane, l'hexane, le cyclohexane, le méthylcyclohexane, l'heptane, le butane ou l'isobutane, le cycloocta-1,5-diène, le dichlorométhane, le dichloroéthane, le chlorobenzène, le dichlorobenzène, le méthanol, l'éthanol, purs ou en mélange. De manière préféré, le solvant est choisi parmi l'hexane, le cyclohexane et le méthylcyclohexane.

Dans le cas où le solvant est un hydrocarbure insaturé, il peut être avantageusement choisi parmi les produits de la réaction d'oligomérisation.

Les exemples suivants illustrent l'invention sans en limiter la portée.

### EXEMPLES

Le taux de solvant est le ratio massique du débit total de solvant injecté sur le débit total d'éthylène injecté dans le procédé.

La distribution en oléfines obtenus par le procédé est donnée en pourcentage de butènes (% C4), d'héxènes (%C6), d'octènes (%C8), de décènes (%C10) et d'oléfines ayant un nombre de carbone égal ou supérieur à 12.

Le %1-C6 correspond à la sélectivité pour en héx-1-ène dans la coupe hexènes.

### Exemples 1 à 5 : Mise en oeuvre du système catalytique en mode continu

### Formulation du système catalytique :

Solution 1 : Dans une éprouvette préparée et sous agitation, le triéthylaluminium (concentration finale = 0,00825 mol/L) est introduit, suivi du chlorure de diethylaluminium (concentration finale = 0,006 mol/L) et du ligand 2,5-diméthylpyrrole (concentration finale = 0,00225 mol/L). Le volume total de la solution est 1,5 L.
Solution 2 : Dans une éprouvette préparée et sous agitation, le précurseur de chrome Cr(2-EH)₃ (xx g, concentration finale = 0,0075 mol/L) et l'anisole (50 eq, 0,03750 mol/L)) sont dilués dans du cyclohexane. Le volume total de la solution est de 1,5 L.

### Mise en oeuvre du système catalytique :

Les solutions du ligand (2,5-diméthylpyrrole) associé aux co-catalyseurs (triéthylaluminium et chlorure de diéthylaluminium) (Solution 1) et de précurseur de chrome (Cr(2-EH)₃) associé au composé aromatique (Anisole et/ou o-Xylène) (Solution 2), diluées dans le cyclohexane (eau mesurée en dessous de 10 ppm), sont introduites dans le réacteur sous contrôle de débit par l'intermédiaire d'un système de pompes. Les 2 lignes d'introduction des solutions sont indépendantes l'une de l'autre. Les points d'injection de ces 2 solutions dans le réacteur se situent directement dans la phase liquide.

L'éthylène (mélangé ou non avec de l'hydrogène à 0,5 % pds) est introduit dans le réacteur sous contrôle de pression. La réaction a lieu dans un réacteur de type CSTR gaz/liquide de 250 mL. Il est équipé d'une canne thermométrique à 5 points permettant la régulation du niveau liquide à différents niveaux, d'un chauffage par bain d'huile (asservi à la température de la phase liquide) et d'une agitation mécanique (de 0 à 1500 tr/min).

La phase liquide contenant l'éthylène dissout, éventuellement l'hydrogène dissout, et les produits formés est évacuée du réacteur via un tube plongeur et une vanne de soutirage, ladite phase est neutralisé, séparé.

La phase gaz est quantifiée et qualifiée par chromatographie en phase gaz (GC), la phase liquide est pesée, neutralisée et qualifiée par GC. Les résultats obtenus sont présentés dans le tableau ci-dessous, les quantités de réactifs sont indiquées en équivalents (noté eq) par rapport à la quantité de chrome mise en oeuvre.

| **Exemples** | **1** | **2** | **3** | **4** | **5** |
|---|---|---|---|---|---|
| Pression (MPa) | 6,0 | 6,0 | 4,0 | 4,0 | 6,0 |
| Température (°C) | 135 | 135 | 115 | 135 | 135 |
| CHARGE | C₂H₄ | C₂H₄/H₂ | C₂H₄/H₂ | C₂H₄ | C₂H₄ |
| Taux de solvant | 62 | 62 | 66 | 75 | 64 |
| Temps de séjour | 0,50 | 0,50 | 0,65 | 0,55 | 0,44 |
| 2,5-DMP | 3 | 3 | 3 | 3 | 3 |
| Et₃Al (eq) | 11 | 10 | 10 | 10 | 11 |
| Et₂AlCl | 8 | 9 | 8 | 9 | 8 |
| Additfi (eq) | Anisole (50) | Anisole (50) | Anisole (50) | o-xylène (500) | o-xylène (500) |
| Conc. Cr réacteur (ppm) | 1,1 | 1,2 | 1,0 | 1,2 | 1,0 |
| Temps de réaction | 16,1 | 15,6 | 6,3 | 14,8 | 17.8 |
| Productivité | 207510 | 202548 | 249249 | 136,587 | 222300 |
| % C4 (%) | 0,0 | 0,0 | 0,0 | 0,0 | 0,0 |
| % C6 (%) | 94,8 | 94,6 | 93,8 | 95,9 | 95.3 |
| % 1-C6 (%) | 99,4 | 99,3 | 99,5 | 99,5 | 99,6 |
| %C8 (%) | 0,2 | 0,2 | 0,2 | 0,2 | 0,2 |
| %C10 (%) | 4,0 | 4,2 | 5,2 | 3,1 | 3,4 |
| % C12+ (%) | 1,0 | 1,0 | 0,7 | 0,9 | 1,0 |

Les exemples 1 à 5 montrent clairement qu'un procédé « in situ » selon la présente invention mettant en oeuvre une composition catalytique comprenant un additif aromatique permet d'obtenir de très bonnes productivités et d'excellentes sélectivités en faveur de l'héx-1-ène.

### Exemple 6 à 8 : Mise en oeuvre du système catalytique en mode batch semi-ouvert

Les exemples 6 à 8 ont été réalisées dans un autoclave en acier inoxydable d'un volume utile de 250 mL muni d'une double enveloppe permettant de réguler la température par circulation d'huile.

Le réacteur est préalablement séché sous vide et mis sous atmosphère d'éthylène.

Solution 1 : Dans un schlenk mis sous atmosphère inerte et sous agitation, en respectant les ratios souhaités, une solution de triéthylaluminium (0.0365 mol/L) dans le cyclohexane est introduite, suivi d'unesolution de chlorure de diéthylaluminium (0.0243 mol/L) dans le cyclohexane et d'une solution de 2,5-diméthylpyrrole (0.0301 mol/L) dans le cyclohexane. Le mélange ainsi obtenu est agité sous argon pendant 15 minutes.

Solution 2 : Le précurseur de chrome Cr(2-EH)₃ (20 µmol) et l'anisole (50 eq) sont dilués dans du cyclohexane. Le volume total de la solution est de 18 mL

Le réacteur est préalablement séché sous vide et mis sous atmosphère d'éthylène. Le cyclohexane est introduit dans le réacteur sous atmosphère d'éthylène puis la solution 1 contenant les Et₃Al (9 - 13 eq/Cr), Et₂AlCl (6 - 10 eq/Cr) et 2,5-DMP (3 à 4 eq/Cr) est ajoutée. La solution 2 est introduite dans le sas d'injection. Le volume total de liquide est de 75 mL. Une fois la température du réacteur portée à la température de test, la solution contenue dans le sas d'injection est injectée dans le réacteur sous pression d'éthylène. La pression est ajustée à la pression de test. La consommation d'éthylène est suivie jusqu'à l'introduction de 35 g d'éthylène. L'alimentation en éthylène est alors coupée et le réacteur refroidi et dégazé. La phase gaz est quantifiée et qualifiée par chromatographie en phase gaz (GC), la phase liquide est pesée, neutralisée et qualifiée par GC.

| Exemple | Et₃Al (eq) | Et₂AlCl (eq) | Cl/Al | Temps (min) | Productivité (g/(gCr.h)) | C4 | %C6 (% 1C6) | %C8 | %C10 | HBP %C12+ |
|---|---|---|---|---|---|---|---|---|---|---|
| **6** | 13 | 6 | 0,32 | 26 | 84400 | 0.6 | 88,3 (98,8) | 0,9 | 5,6 | 4,6 |
| **7** | 11 | 8 | 0,42 | 38 | 50731 | 0.3 | 92,2 (99,2) | 0,5 | 4,6 | 2,4 |
| **8** | 9 | 10 | 0,53 | 55 | 21423 | 0.3 | 94,7 (99,4) | 0,3 | 3,1 | 1,6 |

| | | | | | | | | | | |
|---|---|---|---|---|---|---|---|---|---|---|
| *Conditions opératoires* : Cr(2-EH)₃ (20 µmol); 2,5-DMP (3 eq) ; Anisole (50 eq); Cyclohexane (75 mL); 135 °C, 50 bar | | | | | | | | | | |

Il apparait clairement au vu des exemples 6 à 8 que le procédé selon l'invention mettant en oeuvre une composition catalytique à un ratio précis entre le nombre total d'atomes de chlore apportés par les composants de la composition catalytique et la somme des atomes d'aluminium (CI/AI) dans un procédé d'oligomérisation permet avantageusement de moduler les performances de la composition, notamment en termes de sélectivité et de productivité.

## Revendications

1. Procédé d'oligomérisation d'éthylène comprenant, à une température comprise entre 90 et 190°C, la mise en contact simultanée de l'éthylène avec les composants suivants :
- un précurseur métallique de chrome
- un dérivé pyrrole répondant à la formule générale (I) 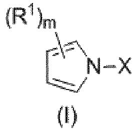 dans laquelle
- R¹ est choisi indépendamment parmi un groupement alkyle en C₁-C₁₅, un groupement C(O)R', un groupement COOR", CCl₃, CF₃, R' étant choisi parmi H, un alkyl en C₁-C₆, un chlore, un brome, un fluor ; R" étant choisi parmi H, un alkyl en C₁-C₆ ;
- m est un entier compris entre 0 et 4 ;
- X est choisi parmi un hydrogène, un lithium, un sodium, un potassium, un césium, ou un atome d'aluminium,
- un composé à base d'aluminium de formule générale AlR²R³R⁴ dans laquelle les groupements R², R³ et R⁴, identiques ou différents, sont choisis indépendamment parmi un hydrogène, les groupements alkyles en C₁-C₂₀, alkoxy en C₁-C₂₀ et aryloxy en C₅-C₃₀,
- au moins un composé halogéné d'aluminium répondant à la formule générale AlₙR⁵ₒYₚ, dans laquelle R⁵ est un groupement alkyle en C₁-C₂₀, Y est un halogène, n est un nombre entier de 1 à 2, o un nombre entier de 1 à 3 et p un nombre entier de 1 à 3 ;
- un additif aromatique,
lesdits composants formant *in situ* une composition catalytique, dans lequel le rapport molaire entre le nombre total d'atome d'halogène apporté par les composants de la composition catalytique et la somme des atomes d'aluminium, noté Altot, apportés par le composé à base d'aluminium et le composé halogéné d'aluminium, noté halo/Altot, est compris entre 0,10 et 3,0.

2. Procédé selon la revendication 1 dans lequel le composé à base d'aluminium et le composé halogéné d'aluminium sont mélangés préalablement à leur mise en contact avec l'éthylène.

3. Procédé selon l'une quelconque des revendications précédentes dans lequel le composé à base d'aluminium et le composé halogéné d'aluminium sont mélangés avant d'être mélangés avec le dérivé pyrrole préalablement à leur mise en contact avec l'éthylène.

4. Procédé selon l'une quelconque des revendications précédentes dans lequel le composé à base d'aluminium et le composé halogéné d'aluminium, le dérivé pyrrole et l'additif aromatique sont mélangés préalablement à leur mise en contact avec l'éthylène.

5. Procédé selon l'une quelconque des revendications précédentes dans lequel le précurseur métallique de chrome et l'additif aromatique sont mélangés préalablement à leur mise en contact avec l'éthylène.

6. Procédé selon l'une quelconque des revendications précédentes dans lequel la température de mise en contact des composants avec l'éthylène est comprise entre 95 et 185°C, de préférence entre 100 et 160°C.

7. Procédé selon l'une quelconque des revendications précédentes dans lequel l'éthylène est en mélange avec de l'hydrogène.

8. Procédé selon l'une quelconque des revendications précédentes dans lequel l'additif aromatique est choisi parmi un éther aromatique et/ou un hydrocarbure aromatique.

9. Procédé selon la revendication 8 dans lequel l'éther aromatique réponds à la formule générale (II) suivante : 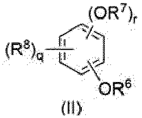 dans laquelle
- R⁶ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R⁷ est choisi parmi un hydrogène, un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- R⁸ est choisi parmi un groupement alkyl en C₁-C₂₀, un groupement cycloalkyle en C₃-C₂₀, un groupement alkényle en C₂-C₂₀, un groupement aryle en C₅-C₂₀ éventuellement substitué par un groupement alkyl en C₁-C₆, ou un groupement aralkyle ;
- q est un nombre entier compris 0 et 4,
- r est un nombre entier égale à 0 ou 1.

10. Procédé selon l'une quelconque des revendications 8 or 9 dans lequel le rapport molaire entre l'éther aromatique et le précurseur métallique à base de chrome, noté éther aromatique/Cr, est compris entre 0,5 et 2000,0.

11. Procédé selon la revendication 8 dans lequel l'hydrocarbure aromatique répondant à la formule générale (III) suivante 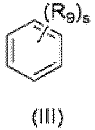 dans laquelle
- R⁹ est choisi indépendamment parmi un groupement alkyle en C₁-C₂₀, un groupement cycloalkyle en C₃-C₂₀,
- s est un entier compris entre 0 et 6.

12. Procédé selon l'une quelconque des revendications 8 ou 11 dans lequel le rapport molaire entre l'hydrocarbure aromatique et le précurseur métallique à base de chrome, noté hydrocarbure aromatique/Cr, est compris entre 5,0 et 6000,0,

## Patentansprüche

1. Verfahren zur Oligomerisation von Ethylen, bei dem man bei einer Temperatur zwischen 90 und 190° Ethylen gleichzeitig mit den folgenden Komponenten in Kontakt bringt:
- einem metallischen Chromvorläufer,
- einem Pyrrolderivat der allgemeinen Formel (I) 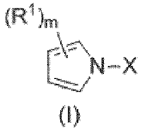 in der
- R¹ unabhängig aus einer C₁-C₁₅-Alkylgruppe, einer C(O)R'-Gruppe, einer COOR"-Gruppe, CCl₃ und CF₃ ausgewählt ist, wobei R' aus H, einem C₁-C₆-Alkyl, einem Chlor, einem Brom und einem Fluor ausgewählt ist; wobei R" aus H und einem C₁-C₆-Alkyl ausgewählt ist;
- m eine ganze Zahl zwischen 0 und 4 ist;
- X aus einem Wasserstoff, einem Lithium, einem Natrium, einem Kalium, einem Caesium oder einem Aluminiumatom ausgewählt ist,
- einer Verbindung auf Aluminiumbasis der allgemeinen Formel AlR²R³R⁴, in der die Gruppen R², R³ und R⁴ gleich oder verschieden sind und unabhängig aus einem Wasserstoff, C₁-C₂₀-Alkyl-, C₁-C₂₀-Alkoxy- und C₅-C₃₀-Aryloxygruppen ausgewählt sind,
- mindestens einer halogenierten Aluminiumverbindung der allgemeinen Formel AlₙR⁵ₒYₚ, in der R⁵ eine C₁-C₂₀-Alkylgruppe ist, Y ein Halogen ist, n eine ganze Zahl von 1 bis 2 ist, o eine ganze Zahl von 1 bis 3 ist und p eine ganze Zahl von 1 bis 3 ist;
- einem aromatischen Additiv,
wobei die Komponenten in situ eine Katalysatorzusammensetzung bilden, in der das Molverhältnis zwischen der Gesamtzahl von den Komponenten der Katalysatorzusammensetzung beigebrachten Halogenatomen und der als Altot bezeichneten Summe der von der Verbindung auf Aluminiumbasis und der halogenierten Aluminiumverbindung beigebrachten Aluminiumatome, das als Halo/Altot bezeichnet wird, zwischen 0,10 und 3,0 liegt.

2. Verfahren nach Anspruch 1, wobei die Verbindung auf Aluminiumbasis und die halogenierte Aluminiumverbindung vor dem Inkontaktbringen mit dem Ethylen gemischt werden.

3. Verfahren nach einem der vorhergehenden Ansprüche, wobei die Verbindung auf Aluminiumbasis und die halogenierte Aluminiumverbindung gemischt werden, bevor sie vor dem Inkontaktbringen mit dem Ethylen mit dem Pyrrolderivat gemischt werden.

4. Verfahren nach einem der vorhergehenden Ansprüche, wobei die Verbindung auf Aluminiumbasis und die halogenierte Aluminiumverbindung, das Pyrrolderivat und das aromatische Additiv vor dem Inkontaktbringen mit dem Ethylen gemischt werden.

5. Verfahren nach einem der vorhergehenden Ansprüche, wobei der metallische Chromvorläufer und das aromatische Additiv vor dem Inkontaktbringen mit dem Ethylen gemischt werden.

6. Verfahren nach einem der vorhergehenden Ansprüche, wobei die Temperatur des Inkontaktbringens der Komponenten mit dem Ethylen zwischen 95 und 185 °C, vorzugsweise zwischen 100 und 160 °C, liegt.

7. Verfahren nach einem der vorhergehenden Ansprüche, wobei das Ethylen in einer Mischung mit Wasserstoff vorliegt.

8. Verfahren nach einem der vorhergehenden Ansprüche, wobei das aromatische Additiv aus einem aromatischen Ether und/oder einem aromatischen Kohlenwasserstoff ausgewählt wird.

9. Verfahren nach Anspruch 8, wobei der aromatische Ether der nachstehenden allgemeinen Formel (II) entspricht: 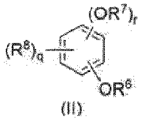 in der
- R⁶ aus einer C₁-C₂₀-Alkylgruppe, einer C₃-C₂₀-Cycloalkylgruppe, einer C₂-C₂₀-Alkenylgruppe, einer C₅-C₂₀-Arylgruppe, die gegebenenfalls durch eine C₁-C₆-Alkylgruppe substituiert ist, oder einer Aralkylgruppe ausgewählt ist;
- R⁷ aus einem Wasserstoff, einer C₁-C₂₀-Alkylgruppe, einer C₃-C₂₀-Cycloalkylgruppe, einer C₂-C₂₀-Alkenylgruppe, einer C₅-C₂₀-Arylgruppe, die gegebenenfalls durch eine C₁-C₆-Alkylgruppe substituiert ist, oder einer Aralkylgruppe ausgewählt ist;
- R⁸ aus einer C₁-C₂₀-Alkylgruppe, einer C₃-C₂₀-Cycloalkylgruppe, einer C₂-C₂₀-Alkenylgruppe, einer C₅-C₂₀-Arylgruppe, die gegebenenfalls durch eine C₁-C₆-Alkylgruppe substituiert ist, oder einer Aralkylgruppe ausgewählt ist;
- q eine ganze Zahl zwischen 0 und 4 ist,
- r eine ganze Zahl mit einem Wert von 0 oder 1 ist.

10. Verfahren nach einem der Ansprüche 8 oder 9, wobei das Molverhältnis zwischen dem aromatischen Ether und der metallischen Vorstufe auf Chrombasis, das als aromatischer Ether/Cr bezeichnet wird, zwischen 0,5 und 2000,0 liegt.

11. Verfahren nach Anspruch 8, wobei der aromatische Kohlenwasserstoff der nachstehenden allgemeinen Formel (III) entspricht: 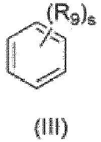 in der
- R⁹ unabhängig aus einer C₁-C₂₀-Alkylgruppe und einer C₃-C₂₀-Cycloalkylgruppe ausgewählt ist,
- s eine ganze Zahl zwischen 0 und 6 ist.

12. Verfahren nach einem der Ansprüche 8 oder 11, wobei das Molverhältnis zwischen dem aromatischen Kohlenwasserstoff und der metallischen Vorstufe auf Chrombasis, das als aromatischer Kohlenwasserstoff/Cr bezeichnet wird, zwischen 5,0 und 6000,0 liegt.

## Claims

1. Ethylene oligomerization process comprising, at a temperature between 90 and 190°C, simultaneously bringing ethylene into contact with the following components:
- a metallic precursor of chromium
- a pyrrole derivative corresponding to the general formula (I) 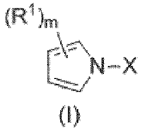 in which
- R¹ is chosen independently from a C₁-C₁₅ alkyl group, a C(O)R' group, a COOR' ' group, CCl₃, CF₃, R' being chosen from H, a C₁-C₆ alkyl, a chlorine, a bromine, a fluorine; R" being chosen from H, a C₁-C₆ alkyl;
- m is an integer between 0 and 4;
- X is chosen from a hydrogen, a lithium, a sodium, a potassium, a caesium, or an aluminium atom,
- an aluminium-based compound of general formula AlR²R³R⁴ in which the groups R², R³ and R⁴, which may be identical or different, are chosen independently from a hydrogen and C₁-C₂₀ alkyl, C₁-C₂₀ alkoxy and C₅-C₃₀ aryloxy groups,
- at least one halogenated aluminium compound corresponding to the general formula AlₙR⁵ₒYₚ, in which R⁵ is a C₁-C₂₀ alkyl group, Y is a halogen, n is an integer from 1 to 2, o is an integer from 1 to 3 and p is an integer from 1 to 3;
- an aromatic additive,
said components forming *in situ* a catalytic composition, wherein the molar ratio between the total number of halogen atoms provided by the components of the catalytic composition and the sum of the aluminium atoms, denoted Altot, provided by the aluminium-based compound and the halogenated aluminium compound, denoted halo/Altot, is between 0.10 and 3.0.

2. Process according to Claim 1, wherein the aluminium-based compound and the halogenated aluminium compound are mixed prior to them being brought into contact with ethylene.

3. Process according to either one of the preceding claims, wherein the aluminium-based compound and the halogenated aluminium compound are mixed before being mixed with the pyrrole derivative prior to them being brought into contact with ethylene.

4. Process according to any one of the preceding claims, wherein the aluminium-based compound and the halogenated aluminium compound, the pyrrole derivative and the aromatic additive are mixed prior to them being brought into contact with ethylene.

5. Process according to any one of the preceding claims, wherein the metallic precursor of chromium and the aromatic additive are mixed prior to them being brought into contact with ethylene.

6. Process according to any one of the preceding claims, wherein the temperature for bringing the components into contact with ethylene is between 95 and 185°C, preferably between 100 and 160°C.

7. Process according to any one of the preceding claims, wherein the ethylene is in a mixture with hydrogen.

8. Process according to any one of the preceding claims, wherein the aromatic additive is chosen from an aromatic ether and/or an aromatic hydrocarbon.

9. Process according to Claim 8, wherein the aromatic ether corresponds to the general formula (II) below: 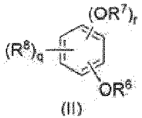 in which
- R⁶ is chosen from a C₁-C₂₀ alkyl group, a C₃-C₂₀ cycloalkyl group, a C₂-C₂₀ alkenyl group, a C₅-C₂₀ aryl group optionally substituted with a C₁-C₆ alkyl group, or an aralkyl group;
- R⁷ is chosen from hydrogen, a C₁-C₂₀ alkyl group, a C₃-C₂₀ cycloalkyl group, a C₂-C₂₀ alkenyl group, a C₅-C₂₀ aryl group optionally substituted with a C₁-C₆ alkyl group, or an aralkyl group;
- R⁸ is chosen from a C₁-C₂₀ alkyl group, a C₃-C₂₀ cycloalkyl group, a C₂-C₂₀ alkenyl group, a C₅-C₂₀ aryl group optionally substituted with a C₁-C₆ alkyl group, or an aralkyl group;
- q is an integer between 0 and 4,
- r is an integer equal to 0 or 1.

10. Process according to either one of Claims 8 and 9, wherein the molar ratio between the aromatic ether and the chromium-based metallic precursor, denoted aromatic ether/Cr, is between 0.5 and 2000.0.

11. Process according to Claim 8, wherein the aromatic hydrocarbon corresponds to the general formula (III) below 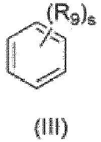 in which
- R⁹ is chosen independently from a C₁-C₂₀ alkyl group, a C₃-C₂₀ cycloalkyl group,
- s is an integer between 0 and 6.

12. Process according to either one of Claims 8 and 11, wherein the molar ratio between the aromatic hydrocarbon and the chromium-based metallic precursor, denoted aromatic hydrocarbon/Cr, is between 5.0 and 6000.0.
